# EUROPÄISCHE PATENTSCHRIFT

(11) **EP 3 679 898 B1**
(45) Veröffentlichungstag und Bekanntmachung des Hinweises auf die Patenterteilung: **18.08.2021**
(21) Anmeldenummer: 19151394.4
(22) Anmeldetag: 11.01.2019
(51) Int. Cl.: A61F 2/30

(54) **IMPLANTAT ZUR STABILISIERUNG UND/ODER FUSION DES ILIOSAKRALGELENKS**
IMPLANT FOR STABILIZING AND/OR FUSION OF THE SACROILIAC JOINT
IMPLANT DESTINÉ À LA STABILISATION ET / OU À LA FUSION DE L'ARTICULATION SACRO-ILIAQUE

(43) Veröffentlichungstag der Anmeldung: 15.07.2020
(73) Patentinhaber: Kraus, Kilian, 97440 Werneck (DE)
(72) Erfinder: Kraus, Kilian, 97440 Werneck (DE)
(74) Vertreter: Schlögl, Markus

(56) Entgegenhaltungen:
- EP-A2- 1 818 025
- WO-A1-2006/020463
- WO-A1-2011/048140
- WO-A1-2019/020690
- DE-A1-102015 121 658
- US-A1- 2001 012 966

## Beschreibung

Die Erfindung betrifft ein Implantat zur Stabilisierung und/oder Fusion des Iliosakralgelenks.

Das Iliosakralgelenk (lat.: Articulatio sacroiliaca) ist ein wenig bewegliches Gelenk, das zwischen dem Kreuzbein (lat.: Os sacrum) und dem linken oder dem rechten Darmbein (lat.: Os ilium) gebildet ist. Bei einem Bruch oder bei Schmerzen, die auf das Iliosakralgelenk zurückzuführen sind, ist es aus medizinischer Sicht oftmals sinnvoll, die Knochenfragmente oder auch die das Iliosakralgelenk bildenden Knochen mittels Implantaten zu fixieren und/oder zu fusionieren. Hierzu sind aus dem Stand der Technik insbesondere stiftähnliche Implantate bekannt, die in die zu verbindenden Knochen bzw. Knochenfragmente eingesetzt werden.

US 7,922,765 B2 beschreibt beispielsweise stiftähnliche Implantate, die insbesondere eine Formgebung, wie etwa einen dreieckigen Querschnitt oder dergleichen aufweisen können, um eine Rotation um die Längsachse des Implantats zu unterbinden.

Um derartige Implantate einsetzen zu können, müssen zunächst entsprechend dimensionierte Ausnehmungen in die zu verbindendenden Knochenfragmente oder Knochen eingebracht werden. Dies erfolgt typischerweise mittels Bohren und/oder Meißeln.

Zur Fixierung des Iliosakralgelenks ist es insbesondere bekannt, mehrere stiftähnliche Implantate nebeneinander vorzusehen, wobei jedes Implantat im Wesentlichen quer zum Iliosakralgelenk verläuft und die aneinanderstoßenden Gelenkflächen überbrückt. Bei einem handelsüblichen Implantatsystem erfolgt die Positionierung der stiftähnlichen Implantate über ein kanüliertes Einführsystem, bei dem die mit einer Durchgangsöffnung versehenen Implantate auf zuvor eingebrachte Führungsstifte geschoben werden. WO2006/020463 A beschreibt ein herkömmliches Schraubimplantat.

Die Erfindung stellt sich zur Aufgabe, ein Implantat zur Stabilisierung und/oder Fusion des Iliosakralgelenks anzugeben, welches eine stabile Fixierung sicherstellt und insbesondere im Rahmen eines minimalinvasiven chirurgischen Eingriffs Verwendung finden kann.

Diese Aufgabe wird gelöst durch ein Implantat zur Stabilisierung und/oder Fusion des Iliosakralgelenks mit den Merkmalen des Patentanspruchs 1.

Vorteilhafte Ausgestaltungen sind Gegenstand der Unteransprüche.

Ein Implantat zur Stabilisierung und/oder Fusion des Iliosakralgelenks, mit einer länglichen, gewendelten äußeren Gestalt, umfasst
- einen konisch zulaufenden inneren Kern (auch: Innenkörper, Innenkern) mit einer Durchgangsöffnung, die sich über die gesamte axiale Länge des Implantats in Axialrichtung längs einer Mittellängsachse erstreckt, und
- zumindest abschnittsweise radial nach außen verlaufende Rippen, die an dem inneren Kern angeordnet sind und sich helikal um zumindest einen Abschnitt des inneren Kerns) in Axialrichtung erstrecken.

Das Implantat hat aufgrund der sich helikal bzw. helixförmig um den inneren Kern und um die Mittellängsachse erstreckenden Rippen eine gewendelte äußere Gestalt. Das Einbringen des Implantats in das Knochenmaterial erfolgt daher im Allgemeinen unter einer Drehbewegung. Eine translatorische Bewegung der mittels des erfindungsgemäßen Implantats verbundenen Knochenpartien in Richtung der Mittellängsachse geht daher mit einer entsprechenden Gegenbewegung in entgegengesetzter Drehrichtung oder mit signifikanten Verdrängung von Knochenmaterial einher. Eine relative Rotation der beiden verbundenen Knochenpartien kann jedoch beispielsweise dadurch effektiv blockiert werden, wenn nicht nur ein einzelnes Implantat zur Fixierung der Knochenpartien eingesetzt wird, sondern mehrere derartig ausgebildete Implantate nebeneinander angeordnet werden. Die Verwendung von mehreren Implantaten mit jeweils gewendelter (auch: gedrallt) äußeren Gestalt erhöht somit auf vorteilhafte Weise die Implantatstabilität insbesondere bei der Fixierung oder Stabilisierung des Iliosakralgelenks.

Der innere Kern erstreckt sich entlang der Mittellängsachse des Implantats und kann im Allgemeinen insbesondere hinsichtlich seines Querschnitts unterschiedlich ausgebildet sein. Gemäß der Erfindung ist der innere Kern um die Mittellängsachse rotationssymmetrisch und insbesondere in Richtung eines ersten Endes konisch zulaufend ausgebildet. Mit anderen Worten verjüngt sich der innere Kern des Implantats zum ersten Ende hin. Auf diese Weise wird das Einsetzen des Implantats vereinfacht und insbesondere die Notwendigkeit, Knochenmaterial in signifikantem Ausmaß vor dem Einsetzen abzutragen, um das Implantat korrekt setzen zu können, verringert. Konisch geformte innere Kerne begünstigen insbesondere das direkte Einbringen des Implantats in Knochenmaterial beispielsweise durch Einschlagen oder Einhämmern. Hierbei ist das erste Ende beim bestimmungsgemäßen Gebrauch des Implantats in Eintriebrichtung zu orientieren, d.h. das Implantat wird mit dem konisch zulaufenden Ende zuerst in das Knochenmaterial eingebracht bzw. eingetrieben. Das Implantat ist dazu ausgebildet, direkt in das Knochenmaterial der zu fixierenden Knochen oder Knochenfragmente eingebracht zu werden. Hierunter soll insbesondere verstanden werden, dass kein oder ein nur geringfügiger Materialabtrag, beispielsweise durch Bohren oder Meißeln, vor dem Einsetzen des Implantats notwendig ist. Ein etwaiger Materialabtrag findet vorzugsweise zur besseren Positionierung des Implantats statt. Das Implantat kann in Ausgestaltungen beispielsweise direkt in den Knochen eingeschlagen werden. Zur korrekten Positionierung des Implantats kann insbesondere ein Führungsstift oder Führungsdraht dienen, der zunächst am Implantationsort eingebracht wird und anschließend in die Durchgangsöffnung des Implantats eingeführt wird.

Die Anzahl der radial hervorstehenden Rippen (auch: Flügel) ist in möglichen Ausgestaltungen unterschiedlich. In bevorzugten Ausgestaltungen sind beispielsweise drei bis fünf radial hervorstehende Rippen am inneren Kern angeordnet.

Die maximale Steigung des konisch zulaufenden inneren Kerns beträgt in Ausgestaltungen lediglich einige wenige Prozent, vorzugsweise in etwa 1%. Bezogen auf den Öffnungswinkel des konisch zulaufenden inneren Kerns bedeutet dies, dass dieser in Ausgestaltungen vorzugsweise maximal einige wenige Grad, besonders bevorzugt weniger als 1°, beispielsweise 0,5° beträgt. Die vorstehend definierte Steigung entspricht dem Tangens des Öffnungswinkels. Die Größe des Öffnungswinkels ist dabei auf die Mittellängsachse bezogen.

In Ausgestaltungen weist der innere Kern im Bereich des ersten Endes eine Zentrierspitze auf, die einen endseitig konisch zulaufenden Abschnitt bildet. Der Öffnungswinkel der Zentrierspitze weicht von dem Öffnungswinkel des konisch zulaufenden inneren Kerns ab und ist im Allgemeinen größer als letzterer. In Ausgestaltungen beträgt der Öffnungswinkel der Zentrierspitze -bezogen auf die Mittellängsachse des Implantats- weniger als 20°, bevorzugt weniger als 15°.

In Ausgestaltungen verjüngen sich die Rippen im Bereich des ersten Endes in Richtung zur Mittellängsachse hin. Auch derartige, optionale Ausgestaltungen dienen dazu, das vorstehend bereits beschriebene direkte Eintreiben des Implantats, insbesondere durch Einschlagen, zu ermöglichen bzw. zu erleichtern.

In Ausgestaltungen weisen die Rippen im Bereich des ersten Endes einen endseitigen Abschnitt mit einem stufenförmigen Profil auf. Das stufenförmige Profil bildet mehrere Schneidkanten, die das Eintreiben des Implantats in das Knochenmaterial weiter vereinfachen.

In Ausgestaltungen weist das stufenförmige Profil mehrere Stufen auf, deren Breiten in Umfangsrichtung in Richtung des ersten Endes zunehmen.

In Ausgestaltungen bilden die Rippen eine rechtshändige Helix und das stufenförmige Profil weist wenigstens eine Stufenkante (auch: Schneidkante) auf, die bezüglich der Axialrichtung und einer Umfangsrichtung derart schräg verläuft, dass beim axialen Eintreiben des endseitigen Abschnitts in Knochenmaterial ein Rechtsdrall bewirkt wird. Alternativ dazu bilden die Rippen eine linkshändige Helix und das stufenförmige Profil weist wenigstens eine Stufenkante auf, die bezüglich der Axialrichtung und einer Umfangsrichtung derart schräg verläuft, dass beim axialen Eintreiben des Implantats in Richtung des endseitigen Abschnitts in Knochenmaterial ein Linksdrall bewirkt wird. Mit anderen Worten ist die zumindest eine Stufenkante des stufenförmigen Profils in Eintriebrichtung entgegen dem Drehsinn der Rippen orientiert, so dass beim axialen Eintreiben des Implantats in Richtung der Mittellängsachse nach dem Prinzip von Actio und Reactio eine Kraftkomponente bewirkt wird, die in einem Drehmoment in Richtung des Drehsinnes der helixförmigen Rippen resultiert. Auf diese Weise wird erreicht, dass das Implantat bereits beim Eintreiben in das Knochenmaterial in eine Drehbewegung versetzt wird, welche das weitere Eintreiben begünstigt.

In Ausgestaltungen sind bzw. ist der innere Kern und/oder die Rippen perforiert ausgeführt und/oder mit einer Kanalstruktur versehen. In bevorzugten Ausgestaltungen weist das Implantat eine Kanalstruktur mit einer Vielzahl von offenen Kanälen auf. Die Kanäle weisen vorzugsweise jeweils eine Querschnittsfläche von 8.000 µm² bis 7.000.000 µm², bevorzugt eine Querschnittsfläche von 50.000 µm² bis 3.100.000 µm², besonders bevorzugt eine Querschnittsfläche von 125.000 µm² bis 570.000 µm² auf. Eine derartig dimensionierte Kanalstruktur ist an die Kapillarwirkung von Blut angepasst und fördert somit das Eindringen von Blut in das Implantat in ausreichender Tiefe. Dadurch wird in vorteilhafter Weise das Verwachsen des Zwischenwirbelimplantats mit anliegenden Knochen unterstützt. In anderen Ausgestaltungen dient die Perforation und/oder die Kanalstruktur dazu, das Implantat im Knochen mittels in das Implantat eingebrachtem, insbesondere eingespritztem Zement, insbesondere Knochenzement oder Knochenersatzmaterial, zu verankern.

In Ausgestaltungen ist die Kanalstruktur wabenförmig, gitter- oder netzartig ausgebildet. Derartige Ausgestaltungen ermöglichen das Eindringen von natürlichem Knochenmaterial und/oder das Einbringen von Knochenersatzmaterial und/oder Füllmaterialien, wie insbesondere Zement, beispielsweise Knochenzement, und sind mechanisch hoch beanspruchbar.

In Ausgestaltungen weist die Kanalstruktur zumindest einen nach außen hin offenen Kanal auf, der eine fluidische Verbindung zur Durchgangsöffnung darstellt. Ein derartig ausgestaltetes Implantat ist insbesondere dazu vorgesehen, nach dem Einsetzen in die zu verbindenden Knochenpartien mit Füllmaterial, wie etwa Zement oder künstlichem Knochenmaterial verfüllt zu werden. Das in die Durchgangsöffnung beispielsweise eingespritzte und aushärtbare Füllmaterial kann insbesondere über den Kanal oder die Kanäle der Kanalstruktur austreten, um das Implantat im Knochenmaterial zu verankern. Derartige Varianten der Erfindung können die Stabilität des Implantats verbessern und eignen sich insbesondere zur Verwendung bei Osteoporosen oder in anderen Fällen, in denen das natürliche Knochenmaterial eine verminderte Tragfähigkeit aufweist oder von einer verminderten Fähigkeit zur Anlagerung von neuem Knochengewebe auszugehen ist.

In Weiterbildung ist das Implantat im Bereich des ersten Endes durch ein Verschlusselement verschließbar. Das Verschlusselement ist beispielsweise eine Art Pfropfen, der in die Spitze des Implantats, insbesondere durch die Durchgangsöffnung, eingeführt werden und dort verankert werden kann. Hierzu sind das Verschlusselement und die Durchgangsöffnung beispielsweise mit Gewinden versehen. Das Verschließen des Endes der Durchgangsöffnung dient maßgeblich dazu, dass das nachträglich eingefüllte Füllmaterial insbesondere über die Kanäle bzw. die Kanalstruktur austritt, um sich mit dem umliegenden, natürlichen Knochenmaterial zu verbinden.

Das Implantat ist dazu vorgesehen, in direktem Kontakt mit Knochenmaterial, insbesondere zumindest abschnittsweise mit dem Kreuzbein und/oder dem Darmbein in Verbindung zu stehen. In vorteilhaften Ausgestaltungen weisen bzw. weist der innere Kern und/oder die Rippen eine Oberflächenstrukturierung, beispielsweise in Form von mehreren hervorstehenden Vorsprüngen, auf. Die Oberflächenstrukturierung weist eine vorgegebene Rauheit auf, die insbesondere im Mikrometerbereich, Submikrometerbereich und/oder in einem Bereich von 10µm bis 100 µm liegt. Dies soll insbesondere Bewegungen des Implantats im eingesetzten Zustand verringern oder unterbinden. Unter dem Mikrometerbereich wird insbesondere der Bereich zwischen 1 µm und 10 µm verstanden. Unter dem Submikrometerbereich wird insbesondere der Bereich zwischen 100nm und 1 µm verstanden. Unter der Wendung, dass die Oberflächenstrukturierung eine vorgegebene Rauheit aufweist, die in einem bestimmten Bereich liegt, soll insbesondere verstanden werden, dass ein die Rauheit der Oberflächenstrukturierung charakterisierender Parameter, wie etwa der flächenbezogene Rauheitswert Sₐ, einen Wert im entsprechenden Bereich annimmt, also beispielsweise für eine im Submikrometerbereich strukturierte Oberfläche einen Wert zwischen 100nm und 1 µm, für eine im Mikrometerbereich strukturierte Oberfläche einen Wert zwischen 1 µm und 10 µm oder für eine im Grob-Mikrometerbereich strukturierte Oberfläche einen Wert zwischen 10 µm und 100 µm annimmt. Die Oberflächenstrukturierung kann insbesondere durch additive oder subtraktive Verfahren erzeugt werden. Insbesondere wenn das Implantat mittels eines generativen Herstellungsverfahrens (auch: additive Herstellung), wie etwa dem selektiven Lasersintern oder dem selektiven Laserschmelzen, hergestellt wird, kann die Oberflächenstrukturierung bereits bei der Herstellung erzeugt werden. Derartig hergestellte Oberflächenstrukturierungen liegen typischerweise im Grob-Mikrometerbereich zwischen 10µm bis 100 µm und können insbesondere eine regelmäßige Struktur in Form von Rillen, Rippen oder Zähnen aufweisen. Zur Herstellung von kleineren Oberflächenstrukturierungen bieten sich vorzugsweise subtraktive oder additive Verfahren an, wie etwa Beschichtungsverfahren oder Auflagerungsverfahren, an, die von den additiven bzw. generativen Herstellungsverfahren zu unterscheiden sind. Zur Oberflächenstrukturierung geeignete subtraktive Verfahren umfassen insbesondere das Ätzen oder das Beaufschlagen der Oberfläche mit einem Strahl von abrasiven Teilchen oder Partikeln. Derartig strukturierte Oberflächen weisen typischerweise statisch verteilte, vorzugsweise homogen verteilte Erhebungen und/oder Senken auf. Die geätzten oder mittels Abrasiv behandelten, metallischen oder nicht-metallischen Oberflächen des Implantats weisen vorzugsweise eine hohe Reinheit insbesondere hinsichtlich Verunreinigungen mit Fremdatomen auf, um hydrophile Eigenschaften der Oberflächen bzw. die Integration des Implantats im Knochen zu begünstigen. Hierzu können insbesondere hochreine Säuren beim Ätzen eingesetzt werden oder ein Ätzvorgang nach der Beaufschlagung der Oberflächen mit abrasiven Partikeln vorgesehen werden, um diese von Strahlmitteln zu reinigen.

In Ausgestaltungen umfasst die Oberflächenstrukturierung zumindest bereichsweise Auflagerungen, die aus einem nicht-metallischen Material, insbesondere aus einem osseokonduktiven und/oder hydrophilen Material besteht. Derartige Auflagerungen sind insbesondere mittels additiver Verfahren oder durch Beaufschlagung der Oberfläche mit nicht-metallischen Teilchen, die beispielsweise aus einem osteokonduktiven und/oder hydrophilen Material bestehen, hergestellt. Die Auflagerungen können beispielsweise von flächigen Beschichtungen gebildet sein, die insbesondere die äußere Oberfläche des Implantats abschnittsweise oder vollständig bildet. In anderen Fällen sind die Auflagerungen durch lokale Einzelkristallauflagerungen oder durch Beaufschlagung der Oberfläche mit Partikeln aus entsprechendem nicht-metallischen Material gebildet. Die Auflagerungen bestehen beispielsweise zumindest zum Teil aus Kalziumphosphat, einem Hydroxylapatit und/oder einer Keramik.

Alternativ oder zusätzlich ist bzw. sind der innere Kern und/oder die Rippen beispielsweise porös, insbesondere offenporig porös ausgebildet, um ein Einwachsen von Knochenmaterial zu ermöglichen. Insbesondere kann die vorstehend beschriebene Kanalstruktur dadurch gebildet sein, dass der Kern und/oder die Rippen porös ausgebildet sind. In anderen Ausgestaltungen sind die Kontaktoberflächen im Wesentlichen glatt ausgebildet.

In Ausgestaltungen weisen die sich helikal um die Mittellängsachse erstreckenden Rippen eine konstante Gewindesteigung auf.

In Ausgestaltungen winden sich die sich helikal über die axiale Länge des Implantats erstreckenden Rippen über einen Winkelbereich von weniger als 180°, bevorzugt über einen Winkelbereich zwischen 45° und 120°, besonders bevorzugt über einen Winkelbereich von etwa 90°, um die Mittellängsachse. Die Gewindesteigung ist somit - im Vergleich zu herkömmlichen Schraubimplantaten, die durch Ausüben eines Drehmomentes in das Knochenmaterial eingetrieben werden - relativ flach, um ein direktes Einschlagen bzw. Einhämmern des Implantats in die zu verbindenden Knochenpartien zu ermöglichen.

In Ausgestaltungen haben die Rippen im Querschnitt senkrecht zur Mittellängsachse eine rechteckige oder trapezförmige Gestalt. Insbesondere haben die Rippen in möglichen Ausgestaltungen im Querschnitt die Gestalt eines gleichschenkligen Trapezes.

In Ausgestaltungen weist die Durchgangsöffnung im Bereich eines zweiten Endes, welches dem ersten Ende gegenüberliegend angeordnet ist, ein Innengewinde zum Einschrauben eines Implantationswerkzeugs auf. Derartige Ausgestaltungen ermöglichen insbesondere das nachträgliche Entfernen des bereits eingesetzten Implantats mittels eines ein entsprechendes Außengewinde aufweisenden Implantationswerkzeugs, welches in das in die Durchgangsöffnung endseitig eingebrachte Innengewinde eingeschraubt werden kann.

In Ausgestaltungen sind die Rippen in Umfangsrichtung regelmäßig zueinander beabstandet. In Ausführungsbeispielen mit zwei radial hervorstehenden Rippen sind diese dementsprechend bevorzugt diametral zueinander angeordnet. In Ausführungsbeispielen mit mehr als zwei Rippen sind diese vorzugsweise regelmäßig um den Umfang des inneren Kerns verteilt angeordnet. Das Implantat weist in derartigen Ausgestaltungen beispielsweise einen sternförmigen Querschnitt auf.

In Ausgestaltungen besteht das Implantat zumindest zum Teil aus einem Metall oder einer Metalllegierung, insbesondere einer Titanlegierung. Bevorzugt sind so genannte Grade-5 Legierungen, insbesondere Ti-6Al-4V, welches sich durch hohe Festigkeit und Beständigkeit auszeichnet. In anderen Ausgestaltungen besteht das Metall oder die Metalllegierung aus Titan, Zirkonium, oxidiertem Zirkonium, Hafnium, Platin, Rhodium, Niobium, medizinischem Edelstahl, Cobalt-Chrom-Stahl oder Tantal.

In Ausgestaltungen steht das Volumen der Rippen zu einem Verdrängungsvolumen im Verhältnis von 1/10 bis 1/2 (andere Schreibweise: 1:10 bzw. 1:2), bevorzugt im Verhältnis von 1/5 bis 1/2 (andere Schreibweise: 1:5 bzw. 1:2), besonders bevorzugt im Verhältnis von etwa 1/3 (andere Schreibweise: 1:3). Das Verdrängungsvolumen ergibt sich hierbei aus der Differenz des Volumens eines Rotationskörpers, der sich durch kontinuierliche Rotation des Implantats um die Mittellängsachse ergibt, und dem Volumen des Implantats. Das Verdrängungsvolumen charakterisiert das Volumen des Knochenmaterials, welches entfernt werden müsste, um das Implantat durch Translation in Richtung der Mittellängsachse zu entfernen. Im Wesentlichen entspricht das Verdrängungsvolumen dem Volumen der Zwischenräume, die in Umfangsrichtung jeweils von den radial hervorstehenden Rippen und in radialer Richtung vom Kern bzw. von dem das Implantat einhüllenden Rotationskörper begrenzt sind. Derartig geometrisch dimensionierte Implantate sind auf die Druckfestigkeit von Knochenmaterial in Abhängigkeit des Materials, aus dem das Implantat besteht, angepasst. Geringere Verhältnisse von maximal 1/5 sind insbesondere bei Implantaten, die zumindest zum Teil aus Titan oder einer Titanlegierung bestehen und mittels eines generativen Fertigungsverfahrens hergestellt sind, besonders bevorzugt, da es sich herausgestellt hat, dass derartig hergestellte Implantate eine verminderte Dauerschwingfestigkeit aufweisen.

Bei Implantaten, deren Radialausdehnungen sich entlang der Mittellängsachse nur wenig verändern, kann das vorstehend spezifizierte Verhältnis von Volumen der Rippen zu Verdrängungsvolumen auch näherungsweise anhand von Querschnittsflächen definiert werden. In diesem Fall entspricht das vorstehend definierte Verhältnis dem Verhältnis der Querschnittsfläche der Rippen (also der Querschnittsfläche des Implantats abzüglich der Querschnittsfläche des inneren Kerns) und dem Flächeninhalt einer den maximalen Querschnittsdurchmesser des Implantats einhüllende Kreisscheibe. Der maximale Querschnittsdurchmesser wird insbesondere von der maximalen radialen Ausdehnung der vom inneren Kern radial abstehenden Rippen definiert. Als Querschnittsfläche wird hier insbesondere die Schnittfläche des Implantats bei einem Querschnitt senkrecht zur Mittellängsachse angesehen. Unabhängig von der konkreten geometrischen Gestalt der Querschnittsfläche ist der Durchmesser der einhüllenden Kreisscheibe stets so gewählt, dass die Querschnittsfläche vollständig innerhalb der einhüllenden Kreisscheibe liegt. Die einhüllende Kreisscheibe ist insbesondere die Kreisscheibe mit dem kleinsten Radius bzw. Durchmesser, bei der die Querschnittsfläche des Implantats vollständig innerhalb der einhüllenden Kreisscheibe liegt. Die radiale Ausdehnung der Querschnittsfläche kann in Ausgestaltungen insbesondere entlang der Mittellängsachse geringfügig variieren. In derartigen Ausführungsbeispielen ist die einhüllende Kreisscheibe bezüglich der Querschnittsfläche mit der maximalen radialen Ausdehnung zu definieren. Insbesondere Implantate der hier beschriebenen Form mit konischen inneren Kernen, deren Öffnungswinkel weniger als einige wenige Grad betragen, können im Rahmen dieser Spezifizierung als Implantate angesehen werden, deren Radialausdehnungen sich entlang der Mittellängsachse nur wenig verändern.

Das vorstehend beschriebene Implantat kann beispielsweise zumindest zum Teil unter Verwendung von herkömmlichen, insbesondere subtraktiven Herstellungsverfahren, hergestellt werden. In diesem Zusammenhang kommen insbesondere Fräsen oder andere abtragende Fertigungsmethoden, wie insbesondere das Laserschneiden und/oder die Laserablation in Betracht. In bevorzugten Ausgestaltungen wird das Implantat zumindest zum Teil, besonders bevorzugt vollständig, mittels eines generativen Herstellungsverfahrens (auch: generatives Fertigungsverfahren, additive Fertigung, 3D-Druck), insbesondere mittels selektivem Laserschmelzen, selektivem Lasersintern, Elektronenstrahlschmelzen oder Fused Filament Fabrication hergestellt.

Die Herstellung mittels derartiger additiver Verfahren ist insbesondere bei Implantaten mit filigranen Strukturen, wie insbesondere der bereits genannten Kanalstruktur vorteilhaft. Additive Herstellungsverfahren sind insbesondere zur Herstellung von Implantaten aus metallischen oder nicht-metallischen Werkstoffen geeignet.

Die Oberfläche des Implantats wird vorzugsweise während der Herstellung in zumindest einem Strukturierungsschritt strukturiert. Der Strukturierungsschritt umfasst beispielsweise subtraktive oder additive Verfahrenstechniken. Subtraktive Techniken in diesem Zusammenhang sind beispielweise abtragende Verfahren. Hierzu zählt insbesondere die Laserablation, das chemische Ätzen von Oberflächen oder die Beaufschlagung der Oberfläche mit einem abrasiven, insbesondere abrasive Teilchen enthaltenden Strahl. Zu additiven Verfahrenstechniken zählen insbesondere solche, die dazu geeignet sind, Material zumindest bereichsweise auf der Oberfläche des Implantats anzulagern, wie etwa Beschichtungsverfahren und/oder Verfahren zur Kristallanlagerung (engl.: Discrete Crystalline Deposition). Zur Ablagerung insbesondere einer flächigen Schicht kann beispielsweise eine Metalloberfläche des Implantats anodisiert werden. Durch die vorstehend genannten additiven oder subtraktiven Maßnahmen kann insbesondere die Oberfläche derart strukturiert werden, dass deren charakteristische Rauheit im Submikrometerbereich, im Mikrometerbereich und/oder im Bereich zwischen 10 µm und 100 µm (Grob-Mikrometerbereich) liegt. In vorteilhafter Weise werden verschiedene subtraktive und/oder additive Verfahrenstechniken derart miteinander kombiniert, dass die Oberfläche des Implantats verschiedene Rauheiten aufweist. So kann beispielsweise zunächst eine grobstrukturierte Oberfläche mit einem charakteristischen Höhenprofil im Bereich zwischen 10 µm und 100 µm durch Abstrahlen mit einem Abrasivpartikel enthaltenden Strahl erzeugt werden, der anschließend durch eine Ätzung oder durch mehrere Ätzungen, insbesondere Säureätzungen, eine Mikrostrukturierung im Mikrometerbereich (etwa im Bereich zwischen 1µm und 3 µm) aufgeprägt wird. Alternativ oder zusätzlich kann beispielsweise eine Strukturierung im Submikrometerbereich durch gezielte Anlagerung von Einzelkristallen, insbesondere von Hydroxylapatit erzeugt werden. Chemische Ätzungen eignen sich insbesondere zum Reinigen der Oberflächen, um insbesondere deren hydrophile Eigenschaften, chemische Aktivität oder Fähigkeiten zur Zellanlagerung zur Bildung von Knochengewebe zu verbessern.

Die vorstehend genannten Strukturierungsschritte erfolgen in Ausgestaltungen bevorzugt unter einer Schutzgasatmosphäre, um Verunreinigungen mit Fremdatomen weitgehend zu vermeiden.

Ein Verfahren zum Implantieren des bereits vorstehend beschriebenen Implantats in den menschlichen oder tierischen Körper umfasst die folgenden Verfahrensschritte:
- Bereitstellen eines Zugangs, insbesondere eines minimalinvasiven Zugangs, zu dem Implantationsort;
- Optionales Abtragen von Knochenmaterial am Implantationsort, insbesondere durch Bohren oder Meißeln;
- Optionales Einsetzen einer Positionierungshilfe, insbesondere eines Führungsstiftes oder Führungsdrahtes, am Implantationsort;
- Ansetzen des ersten Endes des Implantats am Implantationsort, optional unter Einführen des Führungsstifts oder des Führungsdrahtes in die Durchgangsöffnung des Implantats;
- Eintreiben des Implantats in axialer Richtung längs der Mittellängsachse unter Einwirkung einer in Richtung der Mittellängsachse wirkenden Axialkraft, insbesondere durch Einschlagen oder Einhämmern in das Knochenmaterial derart, dass das Implantat die zu verbindenden oder zu stabilisierenden Knochen oder Knochenfragmente zumindest abschnittsweise durchdringt;
- Optionales Entfernen der Positionierungshilfe;
- Optionales Verfüllen des Implantats mit Füllmaterial, insbesondere Zement, Knochenzement oder künstlichem Knochenersatzmaterial, zum Verankern des Implantats im natürlichen Knochenmaterial.

In Ausgestaltung des oben beschriebenen Verfahrens werden die vorstehenden Verfahrensschritte wiederholt, um mehrere Implantate, die jeweils die zu verbindenden Knochenpartien überbrücken, nebeneinander zu setzen. Auf diese Weise wird eine relative Rotation der verbundenen Knochenpartien zueinander effektiv unterbunden und die Stabilität der Verbindung verbessert. Dies ist insbesondere bei einem Eingriff zur Stabilisierung oder Fusion des Iliosakralgelenks vorteilhaft.

In Ausgestaltungen des Verfahrens wird beim Abtragen des Knochenmaterials eine Bohrung in die zu verbindenden Knochen oder Knochenfragmente eingebracht, deren Länge im Wesentlichen der axialen Länge des Implantats entspricht und deren Durchmesser maximal dem Durchmesser des Innenkerns entspricht.

In anderen Ausgestaltungen erfolgt das Einsetzen des Implantats ohne vorherigen Abtrag von Knochenmaterial. Hierbei werden beispielsweise Implantate mit Zentrierspitze ohne Vorbohren in das Knochenmaterial der zu verbindenden Knochenpartien zumindest unter Auswirkung einer längs der Mittelachse gerichteten Axialkraft eingetrieben.

Für eine weitere Beschreibung der Erfindung wird auf die in den Zeichnungsfiguren gezeigten Ausführungsbeispiele verwiesen. Es zeigen in einer schematischen Darstellung:
- Fig. 1: ein Implantat zur Stabilisierung und/oder Fusion des Iliosakralgelenks in einer perspektivischen Darstellung;
- Fig. 2: das Implantat der Fig. 1 in einer weiteren perspektivischen Darstellung;
- Fig. 3: das Implantat der Fig. 1 in einer Seitenansicht;
- Fig. 4: ein erstes Ende des Implantats der Fig. 1 in einer Draufsicht;
- Fig. 5: ein zweites Ende des Implantats der Fig. 1 in einer Draufsicht;
- Fig. 6: Rippen einer möglichen Ausgestaltung des Implantats in einer perspektivischen Ansicht;
- Fig. 7: die Rippen der Fig. 6 in einer Seitenansicht;
- Fig. 8: die Rippen der Fig. 6 in einer in Richtung der Mittellängsachse gerichteten Draufsicht;
- Fig. 9: Rippen einer weiteren möglichen Ausgestaltung des Implantats in einer perspektivischen Ansicht;
- Fig. 10: die Rippen der Fig. 9 in einer Seitenansicht;
- Fig. 11: die Rippen der Fig. 9 in einer in Richtung der Mittellängsachse gerichteten Draufsicht;
- Fig. 12: der innere Kern einer weiteren möglichen Ausgestaltung des Implantats in einer perspektivischen Ansicht;
- Fig. 13: der innere Kern der Fig. 12 in einer Seitenansicht;
- Fig. 14: der innere Kern der Fig. 12 in einer Schnittdarstellung;
- Fig. 15: ein weiteres mögliches Ausführungsbeispiel des Implantats mit zwei diametral zueinander angeordneten Rippen in einer perspektivischen Darstellung;
- Fig. 16: das Ausführungsbeispiel der Fig. 15 in einer weiteren perspektivischen Darstellung;
- Fig. 17: ein weiteres mögliches Ausführungsbeispiel des Implantats mit fünf radial hervorstehenden Rippen in einer perspektivischen Darstellung;
- Fig. 18: das Ausführungsbeispiel der Fig. 17 in einer weiteren perspektivischen Darstellung;
- Fig. 19: ein Ausführungsbeispiel eines Implantats mit Kanalstruktur in einer perspektivischen Darstellung;
- Fig. 20: das Ausführungsbeispiel der Fig. 19 in einer Schnittdarstellung;
- Fig. 21: ein weiteres Ausführungsbeispiel eines Implantats mit Kanalstruktur in einer perspektivischen Darstellung;
- Fig. 22: das Ausführungsbeispiel der Fig. 21 in einer Schnittdarstellung;
- Fig. 23: zwei durch ein Implantat verbundene Teile in einer Seitenansicht;
- Fig. 24: die beiden durch das Implantat verbundenen Teile der Fig. 19 in einer in Richtung der Mittellängsachse gerichteten Draufsicht;

Einander entsprechende oder funktionsgleiche Teile sind in allen Figuren mit den gleichen Bezugszeichen versehen.

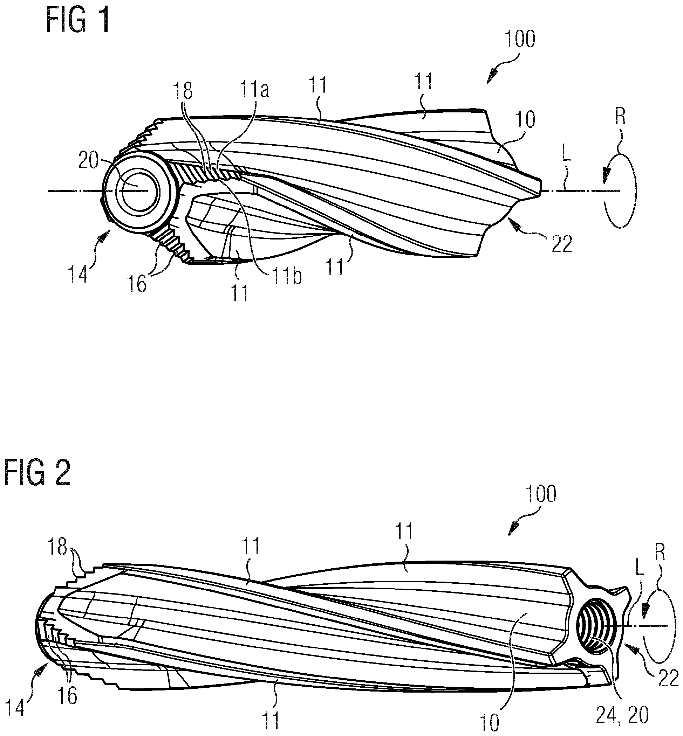

Figuren 1 bis 4 illustrieren ein lediglich exemplarisch und nicht einschränkend aufzufassendes Ausführungsbeispiel des erfindungsgemäßen Implantats 100, welches insbesondere zur Stabilisierung und/oder Fusion des Iliosakralgelenks geeignet ist.

Figur 1 und 2 zeigen das Implantat 100 in perspektivischen Darstellungen. Das dargestellte Implantat 100 besteht aus einem Stück und ist in dem exemplarisch dargestellten Ausführungsform massiv ausgeführt. In anderen Ausführungsbeispielen weist das Implantat 100 beispielsweise eine Kanalstruktur mit mehreren nach außen hin offenen Kanälen auf oder ist aus einem porösem Material gebildet, so dass ein Einwachsen von natürlichem Knochenmaterial ermöglicht ist.

Das Implantat 100 besteht beispielsweise aus einem Metall und ist vorzugsweise mittels eines generativen (auch: additiven) Fertigungsverfahrens, insbesondere mithilfe von selektivem Lasersintern oder selektivem Laserschmelzen gebildet. Die Oberfläche des Implantats 100 ist vorzugsweise hydrophil, um eine Anlagerung von Zellen insbesondere zur Bildung von Knochengewebe zu begünstigen.

Das Implantat 100 ist im Wesentlichen stift- oder bolzenartig ausgeführt und weist eine längliche Gestalt auf, die sich in axialer Richtung längs einer Mittellängsachse L erstreckt. Das Implantat 100 umfasst einen um die Mittellängsachse L rotationssymmetrisch angeordneten und konisch zulaufenden inneren Kern 10, von dem aus sich in radialer Richtung vier Rippen 11 erstrecken, die in Umfangsrichtung regelmäßig beabstandet sind. Im Querschnitt -wie insbesondere in den Figuren 2, 4 und 5 gezeigt- hat das Implantat 100 daher einen sternförmigen Querschnitt.

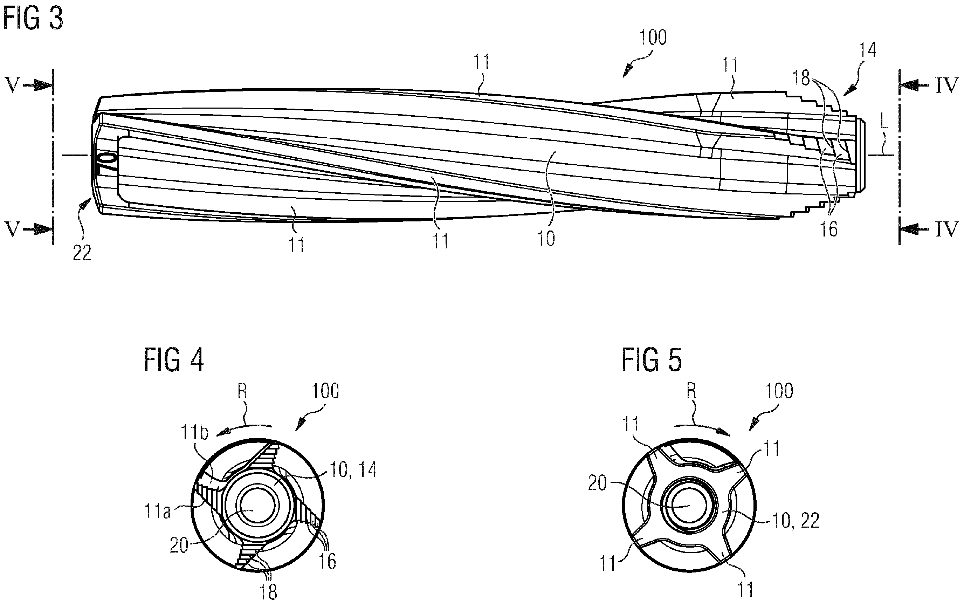

Die Rippen 11 erstrecken sich helikal, also spiralförmig um die Mittellängsachse L und im Wesentlichen über die gesamte axiale Länge des Implantats 100. Insbesondere bilden die Rippen 11 im dargestellten Ausführungsbeispiel eine rechtshändige Helix. Im Bereich eines ersten Endes 14 weisen die Rippen 11 ein stufenförmiges Profil auf, welches mehrere Stufen 16 umfasst. Die Breiten der Stufen 16 in Umfangsrichtung nehmen in Richtung des ersten Endes 14 zu. Im Bereich des ersten Endes 14 ist zudem der innere Kern 10 konisch zulaufend ausgebildet. Das Implantat 100 ist dazu ausgebildet, mit dem ersten Ende 14 zuerst in Knochenmaterial eingetrieben zu werden. Die Ausrichtung der Stufen 16 bzw. von Stufenkanten 18 im Bereich des ersten Endes 14 ist auf die geometrische Gestalt der Rippen 11 ausgelegt: Beim axialen Eintreiben des ersten Endes 14 in Richtung der Mittellängsachse L ins Knochenmaterial bewirken die schräg zur Umfangs- und Axialrichtung verlaufenden Rippen 11 eine Kraftkomponente bzw. ein Drehmoment in Richtung des Umlaufsinnes der helikalen Rippen 11, d.h. einen in Uhrzeigersinn (definiert in Eintrieb- bzw. Vorschubrichtung, also bei einer Blickrichtung entlang der Mittellängsachse L auf das zweite Ende 22, Fig. 5) gerichteten Rechtsdrall R (vgl. insbesondere Figur 4 und 5). Bei Ausführungsbeispielen, deren Rippen 11 jeweils linkhändige Helices bilden, sind die Stufenkanten 18 entsprechend entgegengesetzt orientiert, um beim Eintreiben des Implantats 100 in das Knochenmaterial einen Linksdrall zu erzeugen.

Die Rippen 11 haben in dem sich verjüngenden Bereich nahe des ersten Endes 14 eine asymmetrische Gestalt mit einer flachen ersten Flanke 11a und einer steilen zweiten Flanke 11b. Die flache erste Flanke 11a ist in Richtung des Rechtsdralls R orientiert, um ein Eintreiben des Implantats 100 unter Einwirkung einer Axialkraft weiter zu begünstigen.

Bei Ausführungsbeispielen, deren Rippen 11 linkhändige Helices bilden, sind die Flanken 11a, 11b entsprechend entgegengesetzt angeordnet, d. h. die flachen ersten Flanken 11a sind -bezogen auf eine Blickrichtung entlang der Mittellängsachse L auf das zweite Ende 22 des Implantats 100- entgegen dem Uhrzeigersinn orientiert, um ein Eintreiben des Implantats 100 unter Einwirkung einer Axialkraft weiter zu begünstigen.

Die steile, zweite Flanke 11b kann -wie insbesondere in Fig. 4 gezeigt- eine Krümmung aufweisen.

Das Implantat 100 weist ferner eine Durchgangsbohrung 20 auf, die sich im Wesentlichen über die gesamte axiale Länge des inneren Kerns 10 erstreckt. Beim Einsetzen des Implantats 100 am Implantationsort dient die Durchgangsöffnung 20 der Aufnahme eines zuvor platzierten Führungsstifts oder Führungsdrahtes. An dem zweiten Ende 22 des Implantats 100, welches dem ersten Ende 14 gegenüberliegend angeordnet ist, ist die Durchgangsöffnung 120 mit einem Innengewinde 24 versehen, in das ein Implantationswerkzeug mit einem komplementär ausgebildeten Außengewinde eingeschraubt werden kann. Mit Hilfe des Implantationswerkzeugs kann das bereits eingesetzte Implantat 100 nachträglich entfernt werden.

Das in den Figuren 1 bis 4 exemplarisch dargestellte Ausführungsbeispiel hat eine im Wesentlichen glatte Oberfläche. Alternativ dazu ist vorgesehen, die Oberfläche des Implantats 100 mit einer Oberflächenstrukturierung, insbesondere mit einer Vielzahl von Zähnen oder dergleichen zu versehen, um Bewegungen des Implantats entgegenzuwirken.

Das Implantat 100 ist hohl. In vorteilhaften Ausführungsbeispielen kann der inneren Kern 10 und/oder die Rippen 11 porös ausgeführt sein oder eine Kanalstruktur aufweisen, um ein Einwachsen von natürlichem Knochenmaterial zu ermöglichen. Aufgrund der gewendelten äußeren Gestalt des Implantats 100 sind Bewegungen der durch das Implantat 100 fixierten Knochen bzw. Knochenfragmente im Allgemeinen nur unter einer relativen Drehbewegung um die Mittellängsachse L möglich. Wenn die zu stabilisierenden Knochen oder Knochenfragmente mithilfe von mehreren nebeneinander angeordneten Implantaten 100 fixiert werden, sind derartige Drehbewegungen effektiv blockiert. Durch derartige Maßnahmen kann somit die Stabilität der durch die Implantate 100 vermittelten Verbindung erhöht werden und der Bewegungsspielraum der verbundenen Knochenpartien weitereingeschränkt werden, was insbesondere bei Operationen zur Stabilisierung oder Fusion des Iliosakralgelenks vorteilhaft sein kann.

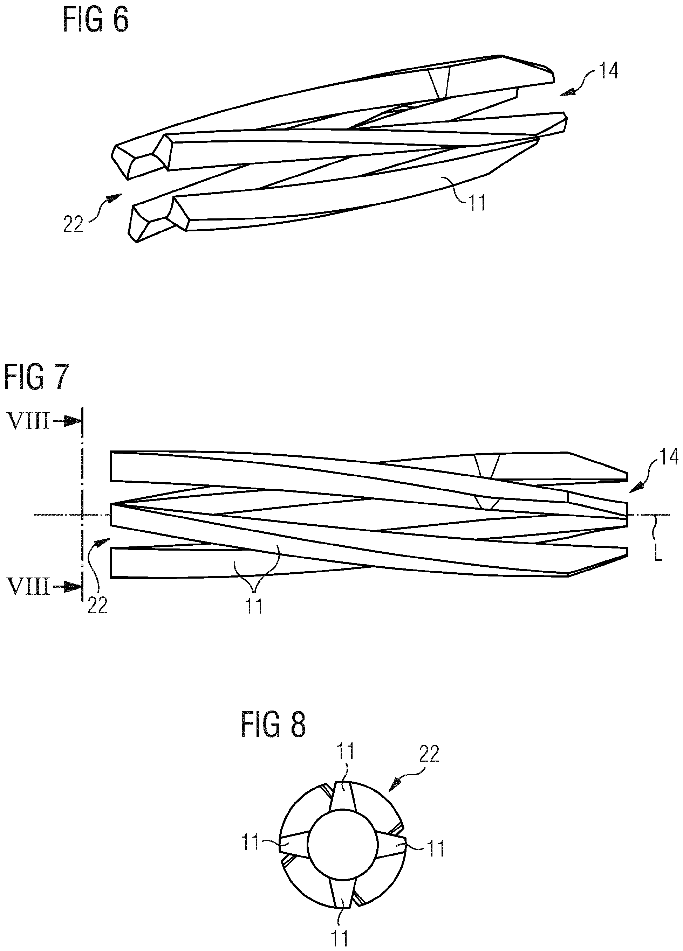

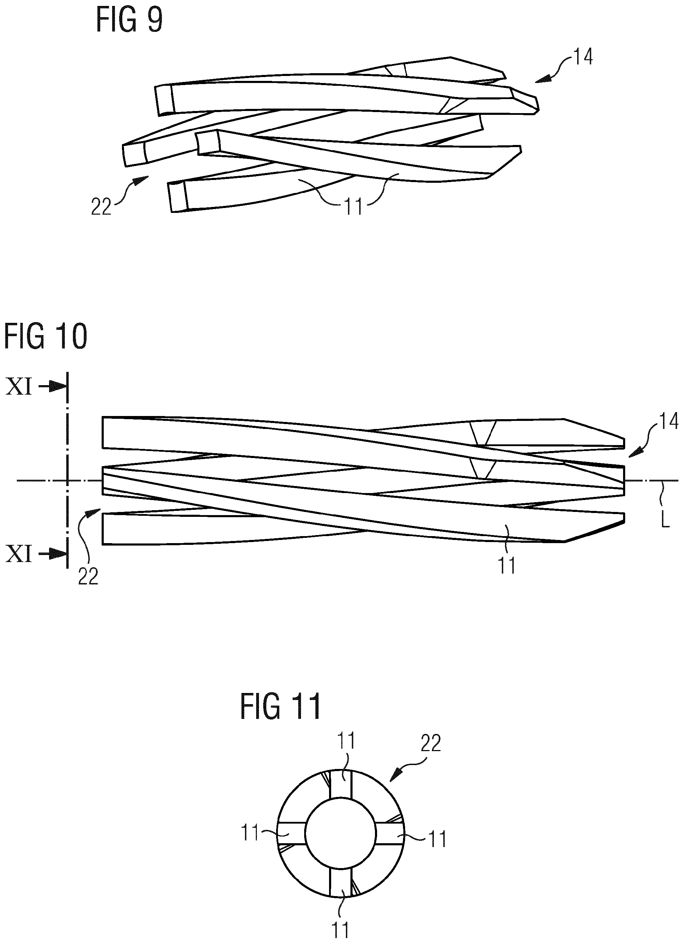

Mögliche Variationen hinsichtlich der Formgebung der Rippen 11 sind insbesondere in Figuren 6 bis 11 illustriert. Die Figuren 6 bis 11 zeigen zur besseren Darstellung der Rippen 11 diese ohne den inneren Kern 10, dessen Form ebenfalls in unterschiedlichen Ausgestaltungen verschieden ausgebildet sein kann.

Figuren 6 bis 8 illustrieren eine mögliche Variation der Rippen 11 in unterschiedlichen Ansichten. Exemplarisch gezeigt ist eine Ausgestaltung mit vier in Umfangsrichtung regelmäßig voneinander beabstandeten Rippen 11, die im Querschnitt (vgl. insbesondere Figuren 6 und 8) trapezförmig ausgebildet sind. Konkret haben die Rippen 10 im Querschnitt die Gestalt eines gleichschenkligen Trapezes. Die Rippen 10 erstrecken sich im Wesentlichen über die gesamte axiale Länge des Implantats und verlaufen dabei spiralförmig um einen Winkelbereich von etwa 90° um die Mittellängsachse L.

Figuren 7 bis 11 illustrieren eine weitere mögliche Variation der Rippen 11 in unterschiedlichen Ansichten. Exemplarisch gezeigt ist eine Ausgestaltung mit vier in Umfangsrichtung regelmäßig voneinander beabstandeten Rippen 11, die im Querschnitt (vgl. insbesondere Figuren 6 und 8) rechteckig ausgebildet sind. Die Rippen 10 erstrecken sich im Wesentlichen über die gesamte axiale Länge des Implantats und verlaufen dabei spiralförmig um einen Winkelbereich von etwa 90° um die Mittellängsachse L.

Die Rippen 11 der in Figuren 6 bis 11 gezeigten Ausführungsbeispiele verjüngen sich im Bereich des ersten Endes 14 zur Mittellängsachse L hin. In dem sich verjüngenden Bereich sind die Rippen 11 glatt ausgeführt. In anderen Ausgestaltungen können die Rippen 11, wie beispielsweise in Figuren 1 bis 4 exemplarisch dargestellt, im Bereich des ersten Endes 14 als ein mehrere Stufen 16 aufweisendes, stufenförmiges Profil ausgebildet sein.

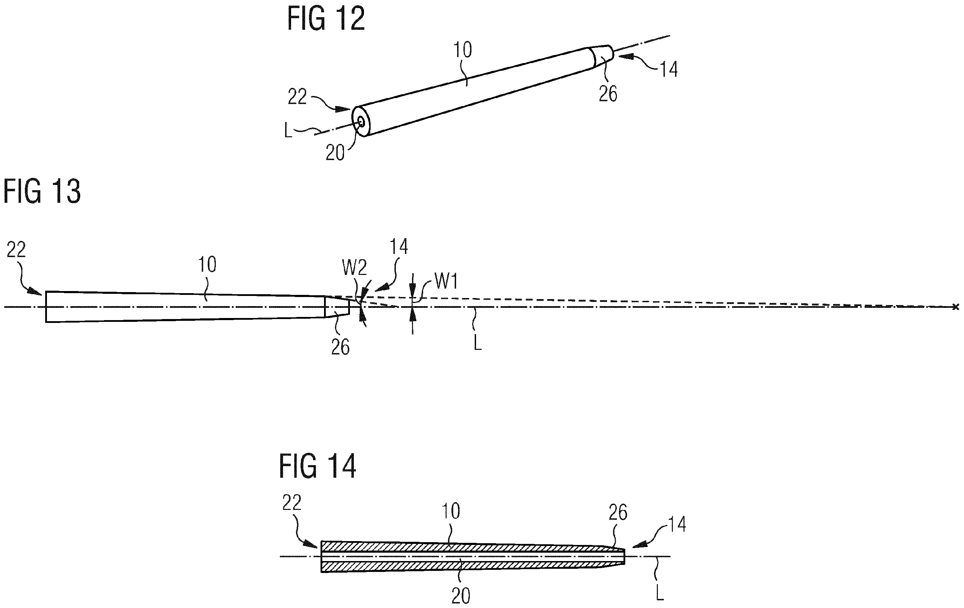

Mögliche Variationen hinsichtlich der Formgebung des inneren Kerns 10 sind insbesondere in Figuren 12 bis 14 in unterschiedlichen Ansichten illustriert. Die Figuren 12 bis 14 zeigen zur besseren Darstellung des inneren Kerns 10 diesen ohne daran angeordnete Rippen 11, die beispielsweise die in Figuren 6 bis 8 oder 9 bis 11 gezeigte Gestalt aufweisen können.

Der innere Kern 10 ist bezüglich der Mittellängsachse L rotationssymmetrisch ausgebildet und läuft in Richtung des ersten Endes 14 konisch zu. Der Öffnungswinkel W1 des konischen inneren Kerns 10 beträgt bezogen auf die Mittellängsachse typischerweise höchstens nur einige wenige Grad, in möglichen Ausführungen sogar weniger als 1°, beispielsweise etwa 0,5°.

Wie in Figuren 12 bis 14 gezeigt, umfasst der innere Kern 10 in möglichen Ausführungsbeispielen einen weiteren konischen Abschnitt mit unterschiedlichem Öffnungswinkel W2, der im Bereich nahe des ersten Endes 14 eine Zentrierspitze 26 bildet. Der Bereich der Zentrierspitze 26 ist beispielsweise identisch mit dem Bereich, in dem die Rippen 11 sich in Richtung der Mittellängsachse L verjüngen. Der Öffnungswinkel W2 der Zentrierspitze ist größer als der Öffnungswinkel W1 des konischen Kerns 10. Bezogen auf die Mittellängsachse L beträgt der Öffnungswinkel W2 beispielsweise weniger als 20°, bevorzugt weniger als 15°. In dem in den Figuren 12 bis 14 lediglich exemplarisch und nicht maßstabsgetreuen dargestellten Beispiel beträgt der Öffnungswinkel W2 der Zentrierspitze 26 in etwa 10°.

Obwohl die Rippen 11 und der Kern 10 in den Figuren 6 bis 14 getrennt dargestellt sind, versteht es sich, dass diese Teile in Ausgestaltungen als ein Stück, ins besondere mit Hilfe eines additiven oder generativen Fertigungsprozesses hergestellt sind.

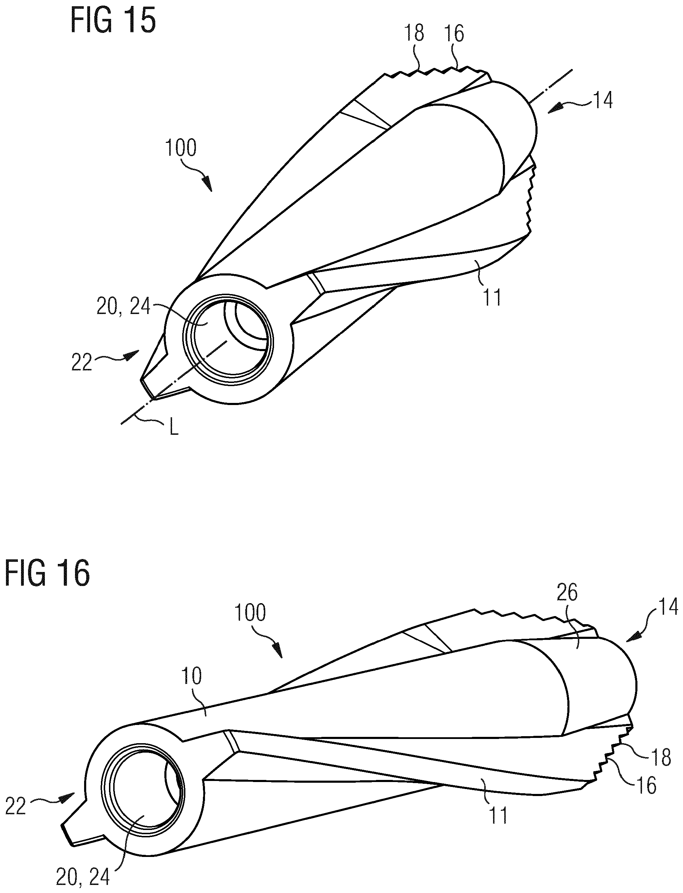

Figuren 15 und 16 zeigen perspektivische Ansichten eines weiteren Ausführungsbeispiels, bei dem lediglich zwei zueinander diametral angeordnete Rippen 11 vorgesehen sind, die vom inneren Kern 10 in radialer Richtung hervorstehen. Die Rippen 11 erstrecken sich über die gesamte axiale Länge des inneren Kerns 11 und insgesamt über einen Winkelbereich von etwa 90° um die Mittellängsachse L. Die Gewindesteigung der Rippen 11 ist somit relativ flach ausgeführt.

In möglichen Ausführungsbeispielen kann die Gewindesteigung der helixförmigen Rippen 11 größer oder kleiner sein. Insbesondere erstrecken sich die Rippen 11 über einen gesamten Winkelbereich von weniger als 180°, beispielsweise über einen Winkelbereich zwischen 45° und 120°.

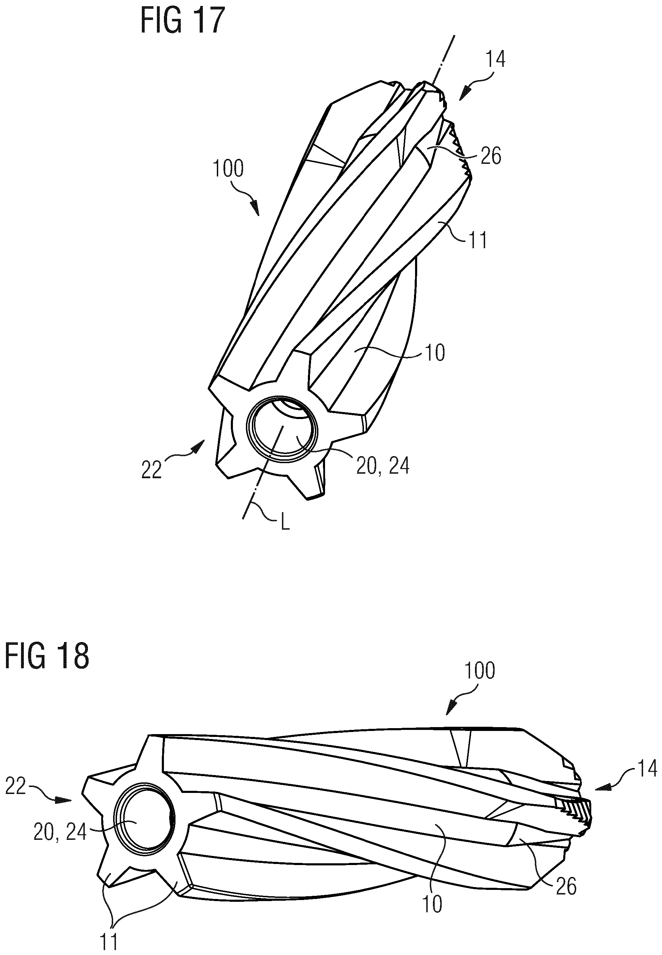

Figuren 17 und 18 zeigen perspektivische Ansichten eines weiteren Ausführungsbeispiels, bei dem fünf radial vom inneren Kern 10 abstehende Rippen 11 vorgesehen sind, die sich spiralförmig um die gesamte axiale Länge des Kerns 10 erstrecken. Dabei überstreicht jede Rippe 11 insgesamt einen Winkelbereich von etwa 70° um die Mittellängsachse L.

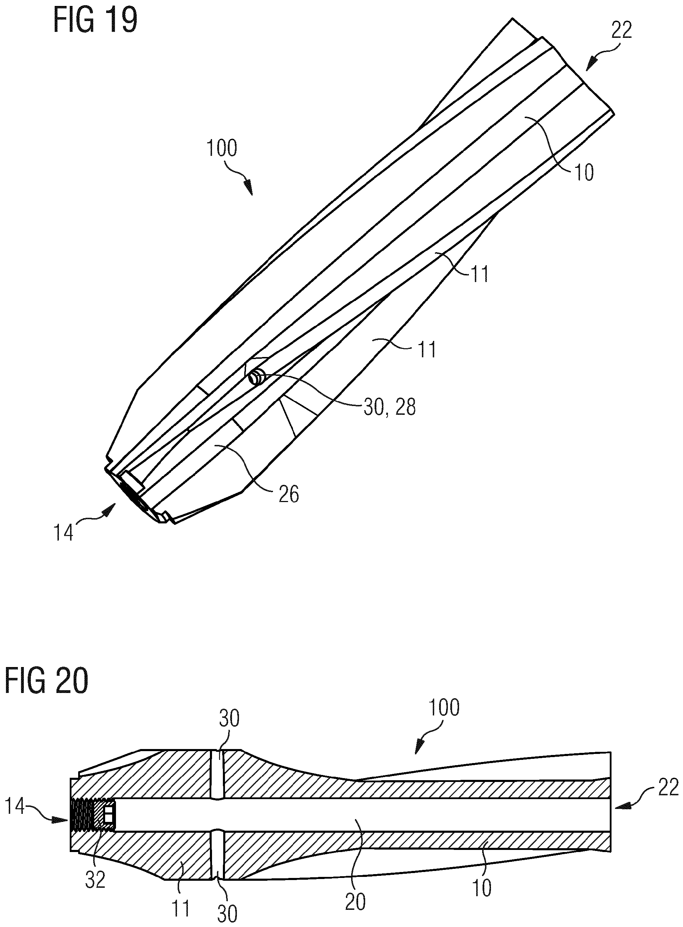

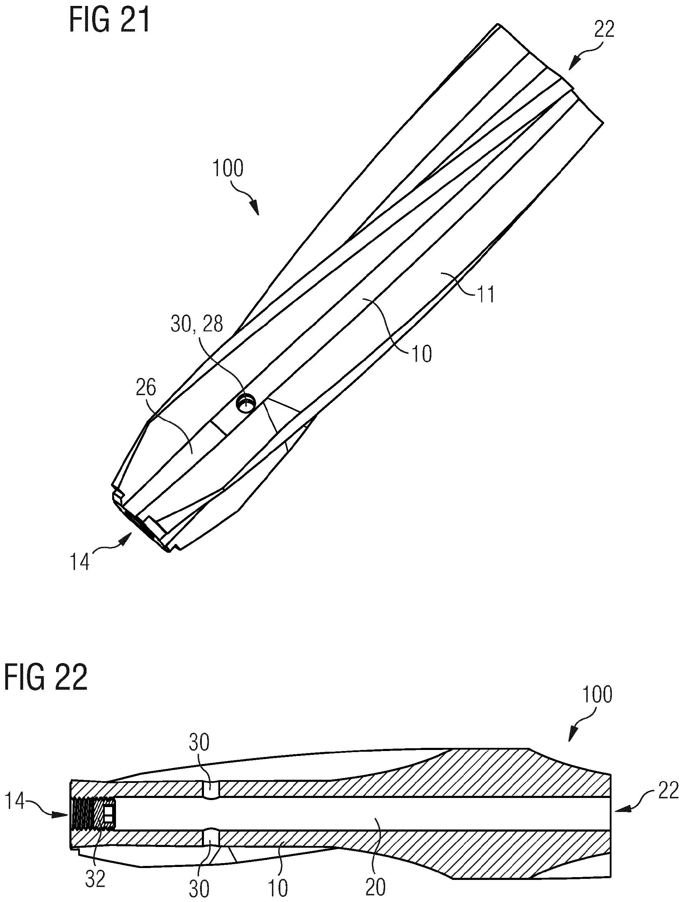

Figuren 19 bis 22 zeigen Ausführungsbeispiele der Erfindung, bei denen das Implantat 100 mit Kanalstrukturen 28 versehen ist, die mehrere Kanäle 30 umfassen. Die Kanäle 30 verbinden die Durchgangsöffnung 20 des Implantats 100 nach außen hin und erstrecken sich in radialer Richtung durch die Rippen 11 und/oder den Kern 10. Die Figuren 19 und 20 zeigen ein Ausführungsbeispiel, bei dem zumindest ein Kanal 30 vorgesehen ist, der sich in radialer Richtung durch die Rippe 11 erstreckt. Die Figuren 21 und 22 zeigen ein weiteres Ausführungsbeispiel, bei dem zumindest ein Kanal 30 vorgesehen ist, der sich in radialer Richtung durch den Kern 10 erstreckt. Im Allgemeinen kann sowohl der Kern 10 als auch die Rippen 11 mit Kanälen 30 versehen sein.

Die Ausführungsbeispiele der Figuren 19 bis 22 sind insbesondere dazu vorgesehen, mit Knochenzement oder künstlichem Knochenersatzmaterial verfüllt werden, um das Implantat 100 am Implantationsort zu verankern. Damit das Füllmaterial durch die radialen Kanäle 30 austritt, ist das Implantat 100 an dem ersten Ende 14 durch ein Verschlusselement 32 verschließbar. Das Verschlusselement 32 weist in dem dargestellten Ausführungsbeispiel hierzu ein Außengewinde auf, das in ein entsprechendes Innengewinde eingreift, das innenseitig in der Durchgangsöffnung im Bereich des ersten Endes 14 eingebracht ist. Nach dem Einsetzen das Implantats 100 in das Knochenmaterial kann das Verschlusselement 32 eingeschraubt werden, um das vordere erste Ende 14 der Durchgangsöffnung 20 zu verschließen.

Bei den in Figuren 15 bis 22 exemplarisch gezeigten Ausführungen des Implantats 100 sind Zentrierspitzen 26 zum verbesserten Einbringen des Implantats 100 insbesondere in Knochenmaterial vorgesehen.

Bei einem Verfahren zum Implantieren des Implantats 100 in den menschlichen oder tierischen Körper wird zunächst ein Zugang, beispielsweise ein minimalinvasiver Zugang, zum Implantationsort bereitgestellt. Gegebenenfalls wird Knochenmaterial am Implantationsort, insbesondere durch Bohren oder Meißeln abgetragen, um beispielsweise das Implantat 100 besser zu positionieren. Dies ist im Allgemeinen jedoch nicht zwingend notwendig, da das Implantat 100 dazu ausgelegt ist, direkt durch Vermittlung einer längs der Mittelachse L wirkenden Axialkraft in das Knochenmaterial eingetrieben zu werden. Gegebenenfalls bietet sich die Verwendung einer Positionierungshilfe, wie etwa eines Führungsstiftes oder Führungsdrahtes, der zuvor am Implantationsort eingebracht wird und anschließend in die Durchgangsöffnung 20 des Implantats 100 eingeführt wird, an.

Das Implantat 100 wird mit seinem ersten Ende 14 zuerst in axialer Richtung unter Einwirkung einer Axialkraft, insbesondere durch Einschlagen oder Einhämmern, in das Knochenmaterial eingetrieben. Das eingesetzte Implantat 100 durchdringt die zu verbindenden oder zu stabilisierenden Knochen oder Knochenfragmente zumindest abschnittsweise und überbrückt insbesondere die Trennfläche zwischen den Knochen bzw. Knochenfragmenten. Eine derartige Situation ist schematisch in Fig. 19 dargestellt, wobei die Knochen lediglich schematisch durch Quader 110, 120 repräsentiert sind. Es versteht sich, dass das Implantat 100 bei einem tatsächlichen chirurgischen Eingriff vorzugsweise vollständig in das Knochenmaterial eingetrieben wird.

Anschließend wird gegebenenfalls die Positionierungshilfe entfernt und das Implantat optional mit Füllmaterial, insbesondere Zement, Knochenzement oder künstlichem Knochenersatzmaterial verfüllt.

Diese Schritte werden vorzugsweise zumindest einmal wiederholt, so dass zumindest zwei Implantate 100 nebeneinander am Implantationsort eingesetzt werden.

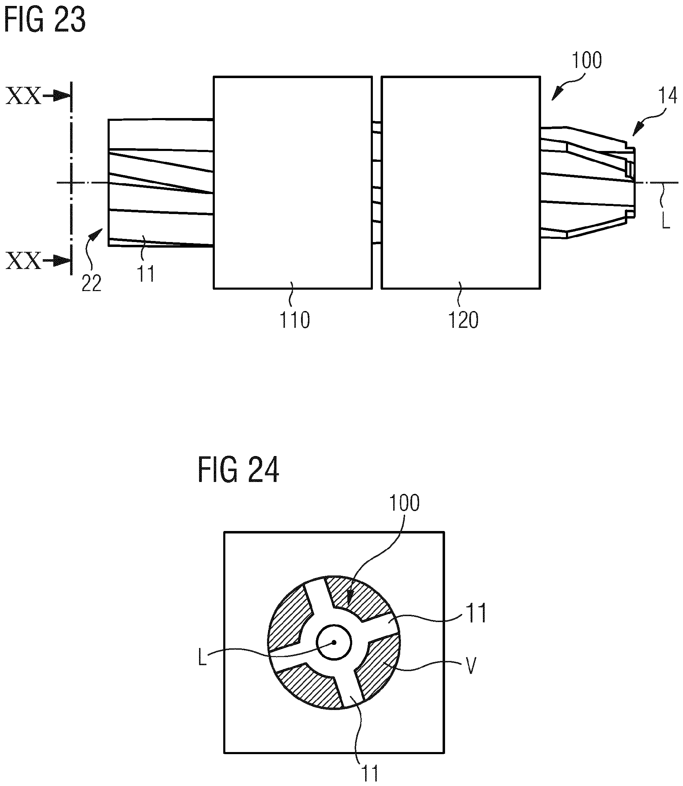

Durch die Verwendung zumindest zweier Implantate 100 zur Fixierung von Knochenpartien wird die Stabilität der Verbindung entscheidend verbessert. Zudem ist die geometrische Auslegung des Implantats 100 in möglichen Ausgestaltungen speziell auf die Verankerung in Knochenmaterial angepasst. Dies soll im Folgenden mit Bezug auf Figuren 24 und 25 erläutert werden.

Falls ein Verfüllen des Implantats 100 mit Füllmaterial erfolgt, so ist vorgesehen, das Implantat 100 gegebenenfalls mit Hilfe des Verschlusselements 32 endseitig zu verschließen, damit das in die Durchgangsöffnung 20 eingefüllte bzw. eingespritzte Füllmaterial über die radial verlaufenden Kanäle 30 austreten kann, um sich so mit dem umliegenden Knochengewebe zu verbinden.

Figuren 24 und 25 illustrieren schematisch eine Situation in einer Seiten- und einer Draufsicht, in der zwei Quader 110, 120 mittels eines erfindungsgemäß ausgestalteten Implantats 100 verbunden sind. Aufgrund der gewendelten äußeren Gestalt des Implantats 100 können diese beiden miteinander verbundenen Quader 110, 120 anschließend nur dann in axialer Richtung zerstörungsfrei auseinander bewegt werden, wenn die Translation in axialer Richtung mit einer relativen Verdrehung der beiden Quader 110, 120 zueinander und um die Längsachse L des Implantats 100 einhergeht. Eine derartige Drehbewegung der Quader 110, 120 zueinander kann jedoch beispielsweise durch Einsetzen eines zusätzlichen Implantats 100 verhindert bzw. blockiert werden, wobei das zusätzlich eingebrachte Implantat 100 ebenfalls Trennfläche zwischen den Quadern 110, 120 überbrückt.

Die geometrische Dimensionierung des Implantats 100 kann speziell auf die Verankerung in Knochenmaterial ausgelegt sein. Es hat sich gezeigt, dass hierfür geeignete Implantate 100 durch das Verhältnis zwischen dem Volumen der Rippen 11 und dem Verdrängungsvolumen V, welches im Wesentlichen dem Volumen der Zwischenräume zwischen den Rippen 11 entspricht, charakterisiert werden können. Bei typischen Materialien für das Implantat 100, wie etwa Metallen, Metalllegierungen, insbesondere Titanlegierungen, beispielsweise Ti-6Al-4V oder andere Metalllegierungen aus Titan, Zirkonium, oxidiertem Zirkonium, Hafnium, Platin, Rhodium, Niobium, medizinischem Edelstahl, Cobalt-Chrom-Stahl oder Tantal, liegt dieses Verhältnis in einem Bereich zwischen 1:2 (1/2) bis 1:10 (1/10), beispielsweise bei etwa 1:3 (1/3).

Das Volumen der Rippen 11 ergibt sich aus dem Volumen des Implantats 100 abzüglich des Volumen des Kerns 10 (vgl. hierzu insbesondere Figuren 6 bis 12).

Das Verdrängungsvolumen V entspricht dem Volumen des Materials, das entfernt werden muss, damit das vollständig in ein Material eingebrachte Implantat 100 durch axiale Translation längs der Mittellängsachse L aus beim Material entnommen werden kann. Das Verdrängungsvolumen V ist in der Querschnittsdarstellung der Fig. 20 schematisch illustriert und entspricht dem Volumen eines Rotationskörpers, der sich durch kontinuierliche Rotation des Implantats 100 um die Mittellängsachse L ergibt, abzüglich dem Volumen des Implantats 100. Im Querschnitt entspricht es dem Volumen der Zwischenräume, die in Umfangsrichtung von den Rippen 11 und in radialer Richtung vom Kern 10 bzw. von einer das Implantat 100 einhüllenden Kreisscheibe K begrenzt sind.

Das insbesondere in den Figuren dargestellte Implantat 100 wird beispielsweise mittels herkömmlicher, insbesondere subtraktiver Herstellungsverfahren, wie etwa dem Fräsen, hergestellt. In Ausgestaltungen wird das Implantat 100 mittels eines generativen Herstellungsverfahrens hergestellt. Beispielsweise wird das Implantat 100 durch selektives Laserschmelzen, selektives Lasersintern, Elektronenstrahlschmelzen oder Fused Filament Fabrication hergestellt. Anschließend wird die Oberfläche des Implantats wird in Ausgestaltungen in zumindest einem Strukturierungsschritt strukturiert. Der Strukturierungsschritt kann insbesondere verschiedene subtraktive oder additive Verfahrenstechniken oder Kombinationen von subtraktiven oder additiven Verfahrenstechniken umfassen. In Ausgestaltungen wird die Oberfläche des Implantats 100 durch Beaufschlagung mit einem Teilchenstrahl aufgeraut und anschießend nass- oder trockenchemisch geätzt. Alternativ oder zusätzlich kann eine Mikrostrukturierung der Oberfläche insbesondere durch Laserablation oder eine Beschichtung der Oberfläche oder ein gezieltes Aufbringen von Materialauflagerungen an der Oberfläche, beispielsweise durch Einzelkristallanlagerungen, erfolgen. Derartige Auflagerungen bestehen beispielsweise aus nicht-metallischen Materialien, wie etwa Kalziumphosphat oder Hydroxylapatit und/oder einer Keramik.

### Bezugszeichen liste

- 10: innerer Kern
- 11: Rippe
- 11a: Flanke
- 11b: Flanke
- 14: erstes Ende
- 16: Stufe
- 18: Stufenkante
- 20: Durchgangsöffnung
- 22: zweites Ende
- 24: Innengewinde
- 26: Zentrierspitze
- 28: Kanalstruktur
- 30: Kanal
- 32: Verschlusselement

- 100: Implantat
- 110: Quader
- 120: Quader

- L: Mittellängsachse
- R: Rechtsdrall
- W1: Öffnungswinkel
- W2: Öffnungswinkel
- V: Verdrängungsvolumen
- K: Kreisscheibe

## Patentansprüche

1. Implantat (100) zur Stabilisierung und/oder Fusion des Iliosakralgelenks, mit einer länglichen, gewendelten äußeren Gestalt, umfassend
- einen konisch zulaufenden inneren Kern (10) mit einer Durchgangsöffnung (20), die sich über die gesamte axiale Länge des Implantats (100) in Axialrichtung längs einer Mittellängsachse (L) erstreckt, und
- zumindest abschnittsweise radial nach außen verlaufende Rippen (11), die an dem inneren Kern (10) angeordnet sind und sich helikal um zumindest einen Abschnitt des inneren Kerns (10) in Axialrichtung erstrecken, wobei die sich helikal über die axiale Länge des Implantats (100) erstreckenden Rippen (11) sich über einen Winkelbereich von weniger als 180° um die Mittellängsachse winden.

2. Implantat (100) nach Anspruch 1, wobei der innere Kern (10) eine Zentrierspitze aufweist, die einen endseitig konisch zulaufenden Abschnitt bildet, wobei ein Öffnungswinkel der Zentrierspitze größer ist als ein Öffnungswinkel des konisch zulaufenden inneren Kerns und vorzugsweise bezogen auf die Mittellängsachse (L) weniger als 20°, besonders bevorzugt weniger als 15° beträgt.

3. Implantat (100) nach Anspruch 1 oder 2, wobei die Rippen (11) sich im Bereich eines ersten Endes (14) in Richtung zur Mittellängsachse (L) hin verjüngen.

4. Implantat (100) nach Anspruch 3, wobei die Rippen (11) im Bereich des ersten Endes (14) einen endseitigen Abschnitt mit einem stufenförmigen Profil aufweisen.

5. Implantat (100) nach Anspruch 4, wobei das stufenförmige Profil mehrere Stufen (16) aufweist, deren Breiten in Umfangsrichtung in Richtung des ersten Endes (14) zunehmen.

6. Implantat (100) nach Anspruch 4 oder 5, wobei die Rippen (11) eine rechtshändige Helix bilden und das stufenförmige Profil wenigstens eine Stufenkante (18) aufweist, die bezüglich der Axialrichtung und einer Umfangsrichtung derart schräg verläuft, dass beim axialen Eintreiben des endseitigen Abschnitts (14) in Knochenmaterial ein Rechtsdrall (R) bewirkt wird oder wobei die Rippen (11) eine linkshändige Helix bilden und das stufenförmige Profil wenigstens eine Stufenkante (18) aufweist, die bezüglich der Axialrichtung und einer Umfangsrichtung derart schräg verläuft, dass beim axialen Eintreiben des endseitigen Abschnitts in Knochenmaterial einen Linksdrall bewirkt wird.

7. Implantat (100) nach einem der vorhergehenden Ansprüche, wobei der innere Kern (10) und/oder die Rippen (11) perforiert ausgeführt sind und/oder mit einer Kanalstruktur versehen sind.

8. Implantat (100) nach Anspruch 7, wobei die Kanalstruktur zumindest einen nach außen hin offenen Kanal aufweist, der eine fluidische Verbindung zur Durchgangsöffnung (20) darstellt.

9. Implantat nach Anspruch 8, wobei die Durchgangsöffnung (20) im Bereich des ersten Endes durch ein Verschlusselement verschließbar ist.

10. Implantat (100) nach einem der vorhergehenden Ansprüche, wobei der innere Kern (10) und/oder die Rippen (11) eine Oberflächenstrukturierung, beispielsweise in Form von mehreren hervorstehenden Vorsprüngen, aufweist, wobei die Oberflächenstrukturierung eine vorgegebene Rauheit aufweist, die insbesondere im Mikrometerbereich, Submikrometerbereich und/oder in einem Bereich von 10µm bis 100 µm liegt.

11. Implantat (100) nach Anspruch 10, wobei die Oberflächenstrukturierung zumindest bereichsweise Auflagerungen umfassen, die aus einem nicht-metallischen Material, insbesondere aus einem osteokonduktiven und/oder hydrophilen Material besteht.

12. Implantat (100) nach einem der vorhergehenden Ansprüche, wobei die sich helikal über die axiale Länge des Implantats erstreckenden Rippen sich über einen Winkelbereich zwischen 45° und 120°, bevorzugt über einen Winkelbereich von etwa 90°, um die Mittellängsachse winden.

13. Implantat (100) nach einem der vorhergehenden Ansprüche, wobei die Durchgangsöffnung (20) im Bereich eines zweiten Endes (22) ein Innengewinde (24) zum Einschrauben eines Implantationswerkzeugs aufweist.

14. Implantat (100) nach einem der vorhergehenden Ansprüche, wobei das Volumen der Rippen (11) und ein Verdrängungsvolumen im Verhältnis von 1/10 bis 1/2, bevorzugt im Verhältnis von 1/5 bis 1/2, besonders bevorzugt im Verhältnis von etwa 1/3, steht, wobei das Verdrängungsvolumen der Differenz des Volumens eines Rotationskörpers, der sich durch kontinuierliche Rotation des Implantats (100) um die Mittellängsachse (L) ergibt, und dem Volumen des Implantats (100) entspricht.

15. Verfahren zum Herstellen eines Implantats (100) nach einem der vorhergehenden Ansprüche, wobei das Implantat (100) mittels eines generativen Herstellungsverfahrens, insbesondere mittels selektivem Laserschmelzen, selektivem Lasersintern, Elektronenstrahlschmelzen oder Fused Filament Fabrication hergestellt wird.

## Claims

1. Implant (100) for stabilising and/or fusing the sacroiliac joint, having an elongated, coiled outer shape, comprising
- a conically tapering inner core (10) having a passage opening (20), which extends across the entire axial length of the implant (100) in the axial direction along a central longitudinal axis (L), and
- ribs (11) running at least sectionally radially outwards, which are arranged on the inner core (10) and extend helically around at least a section of the inner core (10) in the axial direction, wherein the ribs (11) extending helically across the axial length of the implant (100) are wound across an angular region of less than 180° around the central longitudinal axis.

2. Implant (100) according to claim 1, wherein the inner core (10) has a centring point, which forms a conically tapering section on the end side, wherein an opening angle of the centring point is greater than an opening angle of the conically tapering inner core and is preferably less than 20°, particularly preferably less than 15°, in relation to the central longitudinal axis (L).

3. Implant (100) according to claim 1 or 2, wherein the ribs (11) are tapered in the region of a first end (14) in the direction of the central longitudinal axis (L).

4. Implant (100) according to claim 3, wherein, in the region of the first end (14), the ribs (11) have an end-side section having a stepped profile.

5. Implant (100) according to claim 4, wherein the stepped profile has several steps (16), the widths of which increase in the peripheral direction in the direction of the first end (14).

6. Implant (100) according to claim 4 or 5, wherein the ribs (11) form a right-handed helix, and the stepped profile has at least one step edge (18), which runs obliquely in relation to the axial direction and a peripheral direction in such a way that, when axially driving the end-side section (14) into bone material, a right-hand twist (R) is caused or wherein the ribs (11) form a left-handed helix, and the stepped profile has at least one step edge (18), which runs obliquely in relation to the axial direction and a peripheral direction in such a way that, when axially driving the end-side section into bone material, a lefthand twist is caused.

7. Implant (100) according to one of the preceding claims, wherein the inner core (10) and/or the ribs (11) are designed to be perforated and/or are provided with a channel structure.

8. Implant (100) according to claim 7, wherein the channel structure has at least one outwardly open channel, which constitutes a fluidic connection to the passage opening (20).

9. Implant according to claim 8, wherein the passage opening (20) can be sealed by a sealing element in the region of the first end.

10. Implant (100) according to one of the preceding claims, wherein the inner core (10) and/or the ribs (11) have a surface structuring, for example in the form of several prominent protrusions, wherein the surface structuring has a predetermined roughness, which is in particular in the micrometre range, sub-micrometre range and/or in a range of from 10µm to 100µm.

11. Implant (100) according to claim 10, wherein the surface structuring at least regionally comprises coverings, which consist of a non-metallic material, in particular of an osteoconductive and/or hydrophilic material.

12. Implant (100) according to one of the preceding claims, wherein the ribs extending helically across the axial length of the implant are wound around the central longitudinal axis across an angular region of between 45° and 120°, preferably across an angular region of about 90°.

13. Implant (100) according to one of the preceding claims, wherein the passage opening (20) has an inner thread (24) for screwing in an implant tool in the region of the second end (22).

14. Implant (100) according to one of the preceding claims, wherein the volume of the ribs (11) and a displacement volume is at a ratio of from 1/10 to 1/2, preferably at a ratio of from 1/5 to 1/2, particularly preferably at a ratio of about 1/3, wherein the displacement volume corresponds to the difference of the volume of a rotation body, which emerges as a result of continuous rotation of the implant (100) around the central longitudinal axis (L), and to the volume of the implant (100).

15. Method for producing an implant (100) according to one of the preceding claims, wherein the implant (100) is produced by means of a generative production method, in particular by means of selective laser melting, selective laser sintering, electron beam melting or fused filament fabrication.

## Revendications

1. Implant (100) de stabilisation et/ou de fusion de l'articulation sacro-iliaque, ayant une forme extérieure oblongue en limaçon, comprenant
- un noyau (10) intérieur pointant coniquement et ayant une ouverture (20) de passage, qui s'étend sur toute la longueur axiale de l'implant (100) dans la direction axiale le long d'un axe (L) longitudinal médian, et
- des nervures (11), qui s'étendent vers l'extérieur radialement, au moins par endroits, qui sont disposées sur le noyau (10) intérieur et qui s'étendent hélicoïdalement dans la direction axiale autour d'au moins un tronçon du noyau (10) intérieur, les nervures (11) s'étendant hélicoïdalement sur la longueur axiale de l'implant (100) et se tordant sur une plage angulaire de moins de 180° autour de l'axe longitudinal médian.

2. Implant (100) suivant la revendication 1, dans lequel le noyau (10) intérieur a une pointe de centrage, qui forme un tronçon pointant coniquement du côté de l'extrémité, un angle d'ouverture de la pointe de centrage étant plus grand qu'un angle d'ouverture du noyau pointant coniquement et de préférence, rapporté à l'axe (L) longitudinal médian, étant plus petit que 20°, d'une manière particulièrement préférée, plus petit que 15°.

3. Implant (100) suivant la revendication 1 ou 2, dans lequel les nervures (11) se rétrécissent dans la partie d'une première extrémité (14) en direction de l'axe (L) longitudinal médian.

4. Implant (100) suivant la revendication 3, dans lequel les nervures (11) ont, dans la partie de la première extrémité (14), un tronçon du côté de l'extrémité ayant un profil en forme de degrés.

5. Implant (100) suivant la revendication 4, dans lequel le profil en forme de degrés a plusieurs degrés (16), dont la largeur augmente dans la direction du pourtour en direction de la première extrémité (14).

6. Implant (100) suivant la revendication 4 ou 5, dans lequel les nervures (11) forment une hélice tournant à droite et le profil en forme de degrés a au moins un bord (18) de degré qui, par rapport à la direction axiale et une direction de pourtour, s'étend en étant incliné de manière à provoquer, à l'enfoncement axial du tronçon (14), du côté de l'extrémité, dans de la matière osseuse, une torsion (R) à droite ou dans lequel les nervures (11) forment une hélice tournant à gauche et le profil en forme de degrés a au moins un bord (18) de degré, qui s'étend par rapport à la direction axiale et une direction de pourtour en étant incliné, de manière à provoquer, à l'enfoncement axial du tronçon, du côté de l'extrémité, dans de la matière osseuse, une torsion à gauche.

7. Implant (100) suivant l'une des revendications précédentes, dans lequel le noyau (10) intérieur et/ou les nervures (10) sont perforés et/ou sont pourvus d'une structure en canal.

8. Implant (100) suivant la revendication 7, dans lequel la structure en canal a au moins un canal ouvert vers l'extérieur, qui constitue une communication fluidique avec l'ouverture (20) de passage.

9. Implant suivant la revendication 8, dans lequel l'ouverture (20) de passage peut être fermée par un élément de fermeture dans la partie de la première extrémité.

10. Implant (100) suivant l'une des revendications précédentes, dans lequel le noyau (10) intérieur et/ou les nervures (11) ont une structuration de surface, par exemple sous la forme de plusieurs saillies en avancement, la structuration de la surface ayant une rugosité donnée à l'avance, qui est notamment dans le domaine du micron, dans le domaine du sous-micron et/ou dans un domaine de 10µm à 100µm.

11. Implant (100) suivant la revendication 10, dans lequel la structuration de surface comprend, au moins par endroits, des appuis, qui sont en un matériau non métallique, notamment en un matériau ostéo-conducteur et/ou hydrophile.

12. Implant (100) suivant l'une des revendications précédentes, dans lequel les nervures, s'étendant hélicoïdalement sur la longueur axiale de l'implant, se tordent sur une plage angulaire comprise entre 45° et 120°, de préférence sur une plage angulaire d'environ 90°, autour de l'axe longitudinal médian.

13. Implant (100) suivant l'une des revendications précédentes, dans lequel l'ouverture (20) de passage a, dans la partie d'une deuxième extrémité (22), un filet (24) intérieur pour le vissage d'un outil d'implantation.

14. Implant (100) suivant l'une des revendications précédentes, dans lequel le volume des nervures (11) et un volume de déplacement, sont dans le rapport de 1/10 à 1/2, de préférence dans le rapport de 1/5 à 1/2, d'une manière particulièrement préférée dans le rapport d'environ 1/3, le volume de déplacement correspondant à la différence entre le volume d'un corps de révolution, qui s'obtient par rotation continue de l'implant autour de l'axe (L) longitudinal médian, et le volume de l'implant (100).

15. Procédé de fabrication d'un implant (100) suivant l'une des revendications précédentes, l'implant (100) étant fabriqué au moyen d'un procédé de fabrication génératif, notamment au moyen d'une fusion laser sélective, d'un fritage laser sélectif, d'une fusion par faisceau d'électrons ou d'une Fused Filament Fabrication.
